Europäisches Patentamt

European Patent Office

Office européen des brevets

(19)

(11) Veröffentlichungsnummer: **0 258 484 B1**

(12) **EUROPÄISCHE PATENTSCHRIFT**

(45) Veröffentlichungstag der Patentschrift: 06.03.91

(51) Int. Cl.⁵: **A61L 15/16, A61F 13/02**

(21) Anmeldenummer: 86112283.6

(22) Anmeldetag: 05.09.86

(54) **Verwendung einer Klebemasse für einen kohäsiven Verbandstoff.**

(43) Veröffentlichungstag der Anmeldung:
09.03.88 Patentblatt 88/10

(45) Bekanntmachung des Hinweises auf die
Patenterteilung:
06.03.91 Patentblatt 91/10

(84) Benannte Vertragsstaaten:
AT BE CH DE FR GB IT LI LU NL SE

(56) Entgegenhaltungen:
EP-A- 0 035 399
EP-A- 0 080 008
FR-A- 2 452 290
US-A- 4 146 027
US-A- 4 552 802

(73) Patentinhaber: **Karl Otto Braun KG**
**Lauterthalstrasse**
**W-6759 Wolfstein(DE)**

(72) Erfinder: **Langen, Günter Dr. Dipl. Chem**
**Am Herrenacker 7**
**W-6759 Wolfstein/Pfalz(DE)**
Erfinder: **Jung, Harald**
**Im Flürchen 9**
**W-6751 Kreimbach-Kaulbach(DE)**

(74) Vertreter: **Richter, Werdermann & Gerbaulet**
**Neuer Wall 10**
**W-2000 Hamburg 36(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung eines Klebemasse für einen kohäsiven Verbandstoff, dessen einzelne Lagen oder Bindentouren nur auf sich selbst und nicht an der Haut,Haaren und Kleidungsstücken haften; aus einem Flächengebilde aus einem Gewebe, Gewirke, Gestricke oder Vlies für Fixier-, Kompressions-, Stütz- und Entlastungsverbände für medizinische Zwecke, wobei die Klebemasse ein-oder beidseitig auf das Flächengebilde aufbringbar ist.

Bei kohäsiven Verbandstoffen haften beim Umwickeln von verletzten Körperteilen lediglich die sich überlappenden Bindentouren aufeinander; ein Verkleben mit der Haut oder den Haaren tritt jedoch nicht ein. Auch haften beim Abnehmen der Binde keine Rückstände auf der Haut; die Binde läßt sich somit im Gegensatz zu adhäsiv beschichteten Binden schmerzfrei entfernen. Nach der DE-A-561 676 wird als Grundstoff mit kohäsiv haftenden Eigenschaften nicht-koagulierter Naturkautschuk unter Zusatz von nicht näher spezifizierten Alterungsschutzmitteln verwendet.

Nach der DE-A-688 430 ist ein mit Naturlatex im Tauchverfahren beschichtetes Gaze- bzw. Mullband bekannt, wobei die im Naturlatex enthaltenen Proteine im Anschluß an die Beschichtung durch ein Koagulationsverfahren mit Essigsäure und anschließendes Auswaschen entfernbar sein sollen, wodurch eine verbesserte Lagerbeständigkeit und ein Nichtverfärben der in dieser Art imorägnierten Binden erreicht werden soll, wobei die genannten Vorteile auf die Abwesenheit der im Kautschuk enthaltenen Proteine zurückgeführt werden.

Die nach der DE-A-688 430 gefertigten Binden weisen jedoch die bekannten Nachteile von im Tauchverfahren beschichteten Verbandstoffen ,wie weitgehender Verlust der Saugfähigkeit und Luftdurchlässigkeit sowie der Plastizität und des textilen Charakters auf. Darüber hinaus läßt nach umfangreichen Untersuchungen die Verwendung von Naturkautschuk ohne Zusatz von Alterungsmittel keine ausreichende Lagerbeständigkeit erwarten.

Nach der US-A-2,238,878 sind Verfahren zur kohäsiven Ausgestaltung von Mull, Käseleinen, Buttermusselin u.ä. Textilien bekannt, nach denen auf einer oder beiden Oberflächen kleine Fragmente aus Naturkautschuk aufgebracht werden. Dabei sollen die relativ großen Öffnungen im Gewebe aufrechterhalten werden, um eine verbesserte Luftdurchlässigkeit zu erzielen. Die bei diesem Verfahren verwendete GU-Latexdispersion ist bezüglich ihrer Zusammensetzung nicht näher spezifiziert; insbesondere werden keinerlei Angaben über den Zusatz von Alterungsschutzmitteln und sonstigen Hilfsmitteln gemacht, so daß davon ausgegangen werden muß, daß Binden dieser Art die bekannten Nachteile, wie starke Neigung zum Verfärben, Verlust der Kohäsivität und Schmierigwerden durch Alterung bzw. Temperatureinwirkung, aufweisen.

Die US-A-3,575,782 beschreibt ein Verfahren zur Herstellung von elastischen Umhüllungsmaterialien aus nicht gewebten faserigen Gespinsten und hochelastischen Gummi- oder Polyurethan-Fäden unter Verwendung von kohärenten Bindemitteln. Den z.B. aus PES-Fasern bestehenden beiden ungebundenen Vliesstoffbahnen wird eine Anzahl von vorgedehnten Gummifäden so zugeführt, daß diese zwischen den beiden Vliesstofflagen unter Verzug zu liegen kommen. Unter Verwendung von kohärenten Bindemitteln, wie z.B. Naturkautschuk oder synthetischen Polymeren werden die vorgedehnten Gummifäden in dem Laminat so verfestigt, daß beim anschließenden Trocknen und Schrumpfen ein kohäsives, elastisches Laminat entsteht. Die dabei verwendeten gummiartigen Bindematerialien natürlicher oder synthetischer Art bewirken einen schwach klebrigen, adhäsiven Griff, wobei zwar kaum ein Verkleben mit Haut, Haaren oder Kleidungsstücken gegeben ist, aber aufgrund der leicht adhäsiven Eigenschaften weisen Binden dieser Art einen unangenehmen, schmierigen Griff auf. Geht dieser schmierige Griff z.B. durch eine Sterilisation verloren, so ist dies auch verbunden mit dem Verlust der Kohäsivität. Die nach diesem Verfahren gefertigten Vliesstoff-Laminatbinden besitzen aufgrund ihrer Konstruktion einen Bindemittelanteil von mindestens 50%, so daß diese Bänder nicht den im Verbandstoffbereich gewünschten textilen Charakter aufweisen, sondern eher das Verhalten eines Gummibandes besitzen, was sich daraus ergibt, daß die eingearbeiteten PU- oder Gummifäden zu Abschnürungen und unterschiedlichem Flächendruck führen. Als weiterer gravierender Nachteil muß außerdem der Verlust der Luftdurchlässigkeit angesehen werden.

Nach der GB-A-1 297 280 ist ein selbstklebender, nichtgewebter, luftdurchlässiger Vliesstoff aus Stapelfasern bekannt. Die kohäsive Beschichtung des Faservlieses erfolgt mittels einer Mischung aus Naturlatex und Kunstharzlatices, wobei der Anteil an Kunstharz-Latex gegenüber dem Anteil an Naturkautschuk weitaus höher ist. Beide Komponenten dienen hier jedoch in erster Linie der Vliesverfestigung. Durch die partielle Beschichtung mit Klebemasse soll eine ausreichende Festigkeit bei guter Luftdurchlässigkeit erreicht werden, wobei sich die klebenden Eigenschaften je nach Art der Abmischung einstellen.

Die DE-A-1 491 205 beschreibt ein Verfahren zur Herstellung von kohäsiven, elastischen Druckbinden, die aufgrund des partiellen, streifenförmigen Haftmittelauftrages luft- und wasserdampfdurchlässig sind. Bei

EP 0 258 484 B1

den nach diesem Verfahren hergestellten Binden oder Bandagen wird das Gewebeband zwischen tangential angeordneten

Beschichtung swalzen, die mit Aussparungen versehen sind, vorbeigeführt. Durch die Aussparungen der Riffelwalzen wird der gewünschte partielle Auftrag erzielt. Als Haftmischung wird eine Mischung aus trockenem Kautschuklatex, Kaliumoleat, Alterungsschutzmittel und Siliconöl verwendet. Das verwendete Haftmittel muß als Feststoff vor der Beschichtung in einem niedrig siedenden Lösungsmittel, z.B. Benzin, dispergiert werden, was durch die niedrige Trockentremperatur von 35°C weiter bestätigt wird, die zur Anwendung gelangt.

Durch die US-A-4,552,802 ist ein Befestigungsmaterial und insbesondere ein selbstklebendes Produkt in Form eines Blattes, Streifens oder Bandes, ein Verfahren zu seiner Herstellung und die Verwendung als Verbandstoff, Verpackungsmaterial und Befestigungsband bekannt, wobei das selbstklebende Produkt ein Trägermaterial bestrifft, das mit wenigstens einer selbstklebenden Beschichtung versehen ist und aus einem cellularen Material besteht, das mit einer Schicht eines synthetischen oder natürlichen Elastomers beschichtet ist. Dieses Trägermaterial kann ein Blatt oder Band eines cellularen Materials sein, z.B. ein Band oder Gaze oder ein flexibles Blatt (Folie) aus einem synthetischen oder natürlichen Material, welches Perforationen aufweist. Das Elastomer, das als Beschichtungsmasse verwendet und auf das Trägermaterial aufgegeben wird, ist ein synthetisches oder natürliches Elastomer, z.B. Naturkautschuk, das im Handel in Form von hellem Krepp, braunem Krepp oder geräuchertem Krepp erhältlich ist, wobei jedoch die Verwendung von Naturkautschuk des hellen Krepptyps bevorzugt wird. Es ist ebenso möglich ein synthetisches Elastomer zu verwenden, und zwar im besonderen Cis-1,4-Polyisopren, ein Butadienstyrolcopolymer, ein Cis-1,4-Polybutadien, ein Polyoxypropylen, ein Polyoxychlorpropylen, ein Polyacrylat oder ähnliches oder ein Derivat eines Stärkepolymers oder Stärkecopolymers. Als Trägermaterialien kommen hier jedoch keine textilen Flächengebilde, wie Gewebe, Gewirke, Gestricke oder Vliesstoffe zur Anwendung, sondern Trägermaterialien aus cellularen Materialien.

Hinzu kommt, daß als Beschi chtungsmasse bevorzugt Naturkautschuk, Butadi enstyrolcopolmer, Cis-1,4-Polyisopren oder Ci s-1 ,4.Polybutadien verwendet wird. Die zuletzt genannten syntheti schen Elastomerverbindungen weisen die gleiche molekulare Struktur wie Naturkautschuk auf. Insbesondere sind in den Polymermolekülketten noch Doppelbindungen vorhanden, die einer leichten Oxidation zugänglich sind und insofern durch Luft, Sauerstoff, Licht und ähnliche äußere Einf lüsse einer Veränderung unterliegen können, was Eigenschaftsverschlechterungen zur Folge hat . Die hier eingesetzten Elastomeren liegen in Form von Lösungen und nicht als wässrige Di spersionen vor, wobei als Lösungsmittel ein organisches Lösungsmittel, z .B. Dimethyläther, Phenol, Naphtol, Benzin, Toluol od. dgl. verwendet wird. In Lösung gebrachte Elastomere haben jedoch den Nachteil, daß beim Beschi chten des Trägermaterials Lösungsmittel mit in das Trägermaterial gelangt und von diesem aufgesogen wird, denn Lösungen sind homogene, d.h. molekular vertei lte Gemi sche mehrerer Reinststoffe, bei denen eine Komponente, nämlich das Lösungsmittel, im überschuß vorliegt. Beim Auftragen eines in Lösung gebrachten Elastomers wird das Trägermaterial, wie Gewebe od.dgl., durchtränkt mit der Folge, daß die Harzkomponente in die Faser hineingetragen wird, was dazu führt, daß einerseits keine ausreichende Klebewirkung erreicht wird, also der kohäsive Effekt nicht voll erhalten wird, und zum anderen erfolgt ein Verkleben der Fasern unter sich, was z.B. bei Vliesstoffen nicht zu großen Nachteilen führt, weil ja die einzelnen Fasern der Vliesstoffe untereinandder miteinander verklebt werden müssen, jedoch bei Geweben, Gewirken u.dgl. kann es zu einer Verhärtung des Gewebes führen, wodurch die an einen Verhandstoff gestellten Forderungen nicht mehr voll erfüllt werden, insbesondere das Dehnvermögen bzw. das Elastizitätsverhalten wird wesentlich eingeschränkt und die für eine Luftdurchlässigkeit erforderlichen Zwischenräume bzw. Poren können verschlossen werden. Dies ist darauf zurückzuführen,daß eine nicht wässrige Acrylharzlösung das polymere Acrylharz in Form von einzelnen Makromolekülen (Polymerketten) enthält, die monomolekular im Lösemittel verteilt und eingebettet sind. Diese Lösung ist aber ohne Zusatzstoffe stabil, wobei der Feststoffgehalt in % durch die natürliche Sättigungsgrenze eingeschränkt wird. Je höher der Feststoffgehalt, desto höher ist auch die Viskosität der Polymerlösung, so daß bei höheren Feststoffgehalten eine hochviskose, sirupartige Flüssigkeit vorliegt. Eine Acrylharzlösung stellt somit ein homogenes Gemisch, d.h. eine echte chemische Lösung, dar.

Die Verwendung von Lösungen aus einem in organischen Lösungsmitteln gelösten Elastomers führt darüber hinaus dazu, daß die Fasern des Flächengebildes, aus dem der Verbandstoff hergestellt werden soll, nachteilig in ihren Eigenschaften beeinflußt werden.

Die sich beim Einsatz von in Lösungsmitteln gelösten Elastomeren ergebenden Probleme werden daher insofern berücksichtigt, als das Elastomer in einer Menge an einem organischen Lösungsmittel gelöst wird, die ein teigiges und somit hochviskoses Endprodukt entstehen läßt, welches nach bekannten Techniken auf das Trägermaterial aufgebracht wird, wobei es sich bei diesen bekannten Techniken nur um

3

das Aufstreichen, Aufwalzen od.dgl. handeln kann, denn ein Endprodukt mit teigiger Konsistenz läßt sich nicht versprühen und auch nicht durch ein Aerosolverfahren auftragen. Hinzu kommt, daß das eingesetzte Lösemittel beseitigt werden muß. Hierzu wird das bereits beschichtete Material durch einen Trockenkanal mit Heißluft geführt, um das Lösemittel zu verdampfen. Je nach Typ des eingesetzten Elastomers sind auch die entsprechenden organischen Lösemittel einzusetzen. Außerdem bestehen bei der Verarbeitung von Klebemassen auf der Basis organischer Lösungen von vornherein die Nachteile des niedrigen Feststoffgehaltes, d. h. es muß mehr Klebemasse auf den textilen Träger aufgebracht werden, um eine bestimmte kohäsive Klebekraft zu erreichen. Die auf Lösemittel-basis hergestellten Auftragsmassen weisen auch noch den Nachteil auf, daß das Lösemittel nicht vollständig aus einem textilen Trägermaterial entfernt werden kann; darüber hinaus ist es aus Umweltgründen unabdingbar, die Lösemittel durch spezielle Anlagen wieder zurückzugewinnen, was sehr kostenintensiv ist.

Die FR-A-2 452 290 beschreibt ein Verfahren zur Herstellung einer kohäsiven, starren oder elastischen Binde für medizinische Zwecke, wobei das kohäsive Klebeverhalten durch eine Feinstverteilung von Naturlatexpartikeln auf der Oberfläche des textilen Flächengebildes erzielt wird, so daß die Luftdurchlässigkeit, das elastische Verhalten und der textile Charakter des Trägermaterials weitgehend nach der Beschichtung unverändert erhalten bleibt. Bei dem hier verwendeten Klebemittel handelt es sich ausschließlich um eine stabilisierte wässrige Dispersion von Naturkautschuk, welche bislang als Klebemittel zur Herstellung von kohäsiven Verbandstoffen eingesetzt wurde. Solche kohäsiven Verbandstoffe auf Basis Naturkautschuk weisen eine Reihe von Nachteilen auf, welche ihre Verwendungsmöglichkeiten einschränken (Alterungsbeständigkeit, Verfärbungsneigung, nicht vorhandene Sterilisierbarkeit, Lagerbeständigkeit, Oxidationsempfindlichkeit).

Die US-A-4 146 027 betrifft eine Methode zur Wundabdeckung. Dabei handelt es sich nicht um einen kohäsiven Verbandstoff, der nur auf sich selbst, aber nicht auf Haut, Haaren, Kleidungsstücken und fremden Oberflächen haftet, sondern um einen Gegenstand zur Wundabdeckung, der zur Versorgung von verletzten Hautpartien dient. Der Wundverbandstoff selbst besteht aus einem gepreßten oder ungepreßten Schaum, der mit einem flüssigkeits-absorbierenden Trägermaterial verbunden ist, insbesondere Baumwollmull. Diese Schaumschicht ist für Flüssigkeiten durchlässig, so daß Wundexsudat durch die Schaumschicht hindurchtreten kann und von dem mit der Schaumschicht verbundenen absorbierenden Material aufgenommen werden kann. Entsprechend der Zielsetzung, Wundexsudat aufzunehmen, bestehen die hier eingesetzten textilen Trägermaterialien aus cellulosischen Faserstoffen, wie Baumwolle oder Reyon. Der Schaum selbst wird aus Polyacrylsäureestern hergestellt, wobei sowohl Polyacrylsäureäthylester, Polyacrylsäurebutylester als auch Mischungen, d. h. Copolymere von Acrylsäureäthylester mit Methacrylsäuremethylester verwendet werden können. Der gepreßte Schaum soll der Oberfläche des textilen Trägermaterials eine samtartige Textur verleihen, die verhindern soll, daß sich die Fasern untereinander berühren und mit der Wunde in Kontakt kommen. Zusätzlich ist das Copolymer so aufgebaut, daß der Schaum, sogar wenn er gepreßt wird, seine Weichheit und Flexibilität bei niedrigen Temperaturen beibehält und nach dem Trocknen nichtklebend ist, woraus sich ergibt daß diese Wundabdeckung über keine klebenden bzw. haftenden Eigenschaften verfügt.

Die Wundversorgung steht hier im Vordergrund, wobei ein verschäumtes Polymermaterial den Flüssigkeitstransport von der Wunde in eine darüberliegende Saugschicht bewirkt; die Schaumschicht hat keine weitere Funktion, insbesondere keine haftenden Eigenschaften, weder kohäsiv noch adhäsiv.

Im Unterschied zu den mit adhäsiven Klebemassen beschichteten Verbandstoffen, die nahezu auf allen Gebrauchsgegenständen und Materialien haften und einen dauerhaften, aggressiven Tack besitzen, zeichnet sich der rein kohäsive Klebeeffekt dadurch aus, daß die Klebekräfte lediglich innerhalb der Klebemasse wirksam sind und den inneren Zusammenhang der Masse bewirken. Eine mit einer kohäsiven Klebemasse beschichtete Binde haftet demnach nur auf sich selbst, d.h. Lage auf Lage bzw. Bindentour auf Bindentour, aber nicht auf fremden Oberflächen, wie z.B. Haut, Haaren oder Kleidungsstücken. Dieses kohäsive Klebeverhalten wurde bislang überwiegend durch Verwendung von nicht-vulkanisiertem Kautschuk auf Basis Naturlatex eingestellt, wobei entsprechende kohäsive Verbandstoffe nach dem Tauch-, Pflatsch- oder Sprühverfahren unter Verwendung einer meist 60%igen Latex-Dispersion (Zentrifugenlatex) hergestellt werden. In diesem Fall ist es unumgänglich, den Latex vor der Verarbeitung mit Ammoniak zu stabilisieren, was aufgrund der negativen Ionogenität die Möglichkeit der Abmischung mit anderen geeigneten synthetischen Polymeren oder Alterungsschutzmitteln einschränkt. Darüber hinaus ist es bei Verwendung von Naturlatex notwendig, zur Erzielung einer ausreichenden Lagerstabilität (Alterungsbeständigkeit) und zur Vermeidung von Versprödungserscheinungen bzw. Austrocknungsprozessen, wie z.B. sterisch behinderte Phenole, zuzusetzen. Das kohäsive Klebeverhalten kann durch Abmischungen mit geeigneten synthetischen Polymeren derart variiert werden, daß mehr oder weniger stark ausgeprägte Hafteigenschaften vorhanden sind. Als synthetische Polymeren werden vorwiegend kautschuk-ähnliche Latices, wie z.B. Polyisopren,

4

Polybutadien, Butadien-Styrol-, Acrylnitril-Butydien-Copolymere, Polychlorbutadien verwendet.

Bei der Verwendung von Naturlatex als kohäsives Klebemittel müssen eine Reihe von Nachteilen in Kauf genommen werden. Trotz Zusatz von Antioxidantien ist die Lagerbeständigkeit stark eingeschränkt.

Aufgrund der im Kautschukmolekül noch vorhandenen Doppelbindung besteht eine ausgeprägte Reaktionsfähigkeit gegenüber Luftsauerstoff, wobei die Oxidationsprozesse zum Schmierigwerden bzw. zur Versprödung des Kautschuks führen. Diese Effekte werden durch Einwirkung von Licht, Wärme, Strahlung sowie gewisse Chemikalien, wie Schwermetalle u.ä. noch beschleunigt, so daß der Naturkautschuk nach wie vor als ausgesprochen oxidationsempfindlich anzusehen ist. Der Zusatz von Alterungsschutzmitteln bringt eine Reihe von weiteren Nachteilen mit sich. So führen diverse Alterungsschutzmittel bei verschiedenen Personen zu Allergien und Hautreizungen und sind nicht in jedem Fall als toxikologisch unbedenklich anzusehen. Weiterhin neigen einige dieser Antioxidantien zu Verfärbungen, was zu einer Gelb- bzw. Rosafärbung der gesamten Latexmasse führt, so daß der Verbandstoff unansehnlich wird. Dieser Effekt wird teilweise durch die Verpackungs-materialien und Luft noch verstärkt. Es ist weiterhin bekannt, daß hohe Temperaturen zu einer Schädigung der Kautschuk-Klebemasse führen, so daß es zu Schwierigkeiten bei der Hitzesterilisation bzw. Dampfsterilisation entsprechender Verbandstoffe kommt. Auch die Anwendung von anderen Sterilisationsverfahren, wie z.B. Gamma-bzw. Beta-Strahlensterilisationen stoßen auf große Schwierigkeiten, da die Kautschukmoleküle durch die energiereiche Strahlung teilweise gecrackt werden. Der Verbandstoff wird unansehnlich und unbrauchbar (Vergilbung, schmieriger Griff, Versprödung, Verlust der Haftkraft).

Um die bekannten Nachteile des Naturkautschuks und dessen Abmischungen mit kautschukähnlichen synthetischen Polymeren zu umgehen, hat es in der Vergangenheit nicht an Versuchen gemangelt, diese Produktionsnachteile zu beseitigen. So wurde beispielsweise nach verbesserten Antioxidantien und besseren Verpackungsmaterialien gesucht, ohne aber einen durchschlagenden Erfolg erzielt zu haben.

Die Erfindung löst die Aufgabe, eine Klebemasse für einen kohäsiven Verbandstoff gemäß der eingangs beschriebenen Art zu schaffen, der in seinem Eigenschaftsprofil betreffend Luft-, Wasserdampfdurchlässigkeit,Saugfähigkeit, elastisches Verhalten, Wasch-, Lagerbeständigkeit sowie in toxikologischer Hinsicht den bekannten Typen von kohäsiven Verbandstoffen überlegen ist, der sterilisierbar ist und der neben einer hohen Alterungsbeständigkeit auch eine Farbbeständigkeit aufweist. Außerdem soll erreicht werden, daß auch bei einer Lagerung bei erhöhten Temperaturen die Gebrauchseigenschaften einer solchen Binde in keiner Weise negativ beeinflußt werden.

Diese Aufgabe wird durch die Verwendung einer Klebemasse für einen kohäsiven Verbandstoff mit den im Anspruch 1 angegebenen Merkmalen gelöst.

Es hat sich überraschenderweise gezeigt, daß bestimmte, definierte Derivate aus der Klasse der Acrylharzdispersionen das gewünschte rein kohäsive Klebeverhalten aufweisen. Dabei handelt es sich um die Gruppe der vernetzten bzw. unvernetzten Polyacrylsäureester, vorzugsweise Polyacrylsäurebutylester, die als wäßrige Dispersion eingesetzt werden. Diese Dispersionen werden nach diversen, im Nachhinein noch beschriebenen Methoden, auf das Textilsubstrat aufgebracht, so daß ein dem bekannten kohäsiven Bindenstoff entsprechendes Produkt mit jedoch verbesserten Eigenschaften erhalten wird. Die Dispersionen sind in reiner Form verarbeitbar, der Zusatz von Alterungsschutzmitteln bzw. Anti-oxidationsprodukten entfällt, so daß die häufig auftretenden Allergien bei deren Verwendung ausgeschlossen sind. Die beim Kautschuk durch die Anwesenheit von Antioxidantien immer wieder auftretenden Verfärbungen treten bei den verwendeten Acrylsäureestern nicht auf. Insbesondere ist es erstmals gelungen, einen kohäsiven Verbandstoff zu schaffen, der nach den gängigen Sterilisationsmethoden, wie Gas-, Dampf-, Hitze-, Strahlensterilisation, keimfrei gemacht werden kann. Hierdurch ist es möglich, kohäsive Verbandstoffe bereits sterilisiert im OP-Bereich zu verwenden, ohne daß hierbei die Lagerzeit eine Rolle spielt. Neben einer hohen Sterilisierbarkeit zeichnet sich der Verbandstoff durch eine hohe Alterungsbeständigkeit und Farbbeständigkeit aus.

Vorteilhafte Ausgestaltungen der Erfindung sind in den Unteransprüchen gekennzeichnet.

Vorteilhafterweise besteht die Klebemasse aus

1.000 g  vernetztem Polyacrylsäurebutylester in Form einer 50%igen wäßrigen Dispersion,

2 g  Alkylphenylpolyglykoläther als Netzmittel und

10 g  Acrylharzschlichte als Schaumstabilisator.

Des weiteren kann die Klebemasse auch aus 1.000 g unvernetztem Polyacrylsäurepropylester in Form einer 60%igen wäßrigen Dispersion bestehen.

Außerdem sieht die Erfindung eine Klebemasse vor, die aus

100 Teilen  unvernetztem Polyacrylsäurebutylester in Form einer 50%igen wäßrigen Dispersion und

1 Teil  Carboxymethylcellulose besteht.

Ein kohäsiver Verbandstoff, dessen einzelne Lagen oder Bindentouren nur auf sich selbst und nicht an der Haut, Haaren und Kleidungsstücken haften und der aus einem Gewebe, Gewirke, Gestricke oder Vlies für Fixier-, Kompressions-, Stütz- und Entlastungsverbände für medizinische Zwecke besteht, wird in der Weise hergestellt, daß die Klebemasse aus einer Acrylharzdispersion, insbesondere aus einem vernetzten oder unvernetzten Polyacrylsäureester oder einem Polyacrylsäurebutylester mit oder ohne Zusatz eines Netzmittels und/oder von Schaumstabilisatoren ein- oder beidseitig auf dem Gewebe, Gewirke, Gestricke oder Vlies des Verbandstoffes aufgetragen wird. Die Klebemasse wird aus einer verschäumten Axrylharzdispersion aus

1.000 g vernetztem Polyacrylsäurebutylester in Form einer 50%igen wäßrigen Dispersion,
2 g Alkylphenylpolyglykoläther als Netzmittel und
10 g Acrylharzschlichte als Schaumstabilisator

mittels eines Walzenrakels in gleichmäßiger Verteilung auf eine der beiden Oberflächen des aus Schuß und Kette oder aus Kette als Gewirke oder Gestricke oder aus einem Vlies bestehenden Flächengebilde aufgebracht, wobei die aus

1.000 g vernetztem Polyacrylsäurebutylester in Form einer 50%igen wäßrigen Dispersion,
2 g Alkylphenylpolyglykoläther als Netzmittel und
10 g Acrylharzschlichte als Schaumstabilisator

bestehende Masse in einem Schaummixer im Verhältnis 1:5 bei einem Schaumgewicht von 200 g/Liter verschäumt und die Spalthöhe des Rakels so eingestellt wird, daß 20 g Schaum/m$^2$ Gewebe, Gewirke, Gestricke oder Vlies einseitig aufgebracht und anschließend das beschichtete Gewebe, Gewirke, Gestricke oder Vlies bei 130° C getrocknet wird, wobei der Schaum zerfällt und eine vollflächige mikroporöse Oberflächenbedeckung erhalten wird, wobei dann auch die noch nicht beschichtete Rückseite des Flächengebildes in gleicher Weise beschichtet werden kann.

Ferner kann der kohäsive Verbandstoff derart hergestellt werden, daß ein etwa 90 g/m$^2$ Flächengewicht aufweisendes Gewebe, Gewirke, Gestricke oder Vlies, insbesondere längselastische Fixierbinde, beidseitig mit etwa 8 g/m$^2$ Seite mit 100 g unvernetztem Polyacrylsäurepropylester in Form einer 60%igen wäßrigen Dispersion unverdünnt nach dem Aerosolverfahren bzw. durch Verdüsung nach dem Airlessverfahren mikropunktuell in statistischer Verteilung beschichtet wird, wobei die Auflagenmenge über den Pumpendruck und die Fördermenge so gewählt wird, daß pro Gewebeseite eine Auflagenmenge von 8 g bis 10 g/m$^2$ Dispersion aufgebracht wird.

Des weiteren kann auch ein etwa 350 g/m$^2$ Flächengewicht aufweisendes Gewebe, Gewirke, Gestricke oder Vlies, insbesondere längselastische Kompressionsbinde, mit dauerelastischen Polyurethanfäden beidseitig mit etwa 15 g/m$^2$/Seite einer Masse aus

100 Teilen unvernetztem Polyacrylsäurebutylester in Form einer 55%igen wäßrigen Disperion und
1 Teil Carboxymethylcellulose

mittels Siebdruck mikropunktuell kohäsiv beschichtet wird, wobei die verdickte, wäßrige Acrylharzdispersion unter Verwendung beispielsweise einer 25 mesh Schablone mit Lochmusterstruktur auf die Bahn des Gewebes, Gewirkes, Gestrickes oder Vlieses aufgedruckt wird, so daß ein geordnetes Punktmuster auf der Oberfläche des Flächengebildes entsteht, wobei nach dem Trocknen der einseitig beschichteten Gewebebahn in gleicher Weise die Rückseite der Gewebebahn mit der Acrylharzdispersion aus

100 Teilen unvernetztem Polyacrylsäurebutylester in Form einer 55%igen wäßrigen Dispersion und
1 Teil Carboxymethylcellulose

beschichtet wird.

Die mit dem kohäsiven Verbandstoff erzielten Produktvorteile sind auf die Unempfindlichkeit und die Beständigkeit der verwendeten Acrylsäureester gegenüber energiereicher Gamma- bzw. Betastrahlung, Licht, Hitze, Dampf bzw. ETO, zurückzuführen. Es hat sich weiterhin gezeigt, daß Kompressionsbinden, kohäsiv ausgestaltet mit Acrylsäureester, eine deutlich verbesserte Waschbeständigkeit besitzen, so daß eine mehrfache Verwendung möglich ist.

T A B E L L E   I

| Klebemasse | Kautschuk | Rezeptur nach Beispiel 1 | Rezeptur nach Beispiel 2 |
|---|---|---|---|
| Auflagemenge trocken | 20 g/m$^2$ | 20 g/m$^2$ | 16 g/m$^2$ |
| Haftkraft | 70 cN/cm | 78 cN/cm | 38 cN/cm |
| Haftkraft n. 3 J.Alterg. | 60 cN/cm | 77 cN/cm | 40 cN/cm |
| Haftkraft n. 6 J.Alterg. | 31 cN/cm | 75 cN/cm | 38 cN/cm |

| Klebemasse | Kautschuk | Rezeptur nach Beispiel 1 | Rezeptur nach Beispiel 2 |
|---|---|---|---|
| Alterungstest (Lagerbeständigkeit) | 5 Jahre | 15 Jahre | 15 Jahre |
| Waschbeständigkeit | 1 x | 5 x | 7 x |
| Dampfsterilisation | +− | + | + |
| Gassterilisation | + | + | + |
| Hitzesterilisation | − | + | + |
| Strahlensterilisation | − | + | + |

+   möglich        −   nicht   möglich

Die Auflagemenge trocken wird über die Differenz der Flächengewichte des beschichteten und unbeschichteten textilen Trägergewebes ermittelt.

Unter Haftkraft wird diejenige Kraft verstanden, die zur Trennung zweier aufeinanderklebender, kohäsiver Bindenabschnitte, bezogen auf eine Länge von 10 cm und einer Breite von 1 cm, notwendig ist.

Zur Durchführung der Messung wird die Binde auf eine Länge von 10 cm gefaltet und dann mit einer Walze der Masse 5 kg, die eine Temperatur von 37° C aufweist, durch 45 Rollbewegungen innerhalb einer Minute zusammengewalzt. Die Bindenenden werden anschließend in eine KraftDehnungs-Prüfmaschine eingespannt und bis zur völligen Trennung der aufeinanderhaftenden Flächen auseinandergezogen. Durch Integration über die Kraft-Dehnungs-Kurve kann über die Haftarbeit die mittlere Haftkraft in der Einheit cN/cm berechnet werden. Entsprechende Werte der Haftkraft werden nach künstlicher Alterung der kohäsiven Binden ermittelt. Die Durchführung der künstlichen Alterung erfolgt entsprechend DIN 53 896, Prüfung von Textilien "Künstliche Alterung durch erhöhte Temperatur".

Die Binden werden bei 70° C mehrere Tage im Wärmeschrank gelagert. Nach der Formel

$$\frac{a - 1}{2} = X$$

kann aus der Zahl der Lagertage a die Dauer der simulierten Alterung X in Jahren ermittelt werden. Zur Überprüfung der Waschbeständigkeit werden die kohäsiven Binden 30 Minuten bei 60° C mit 5 g/l Vollwaschmittel bei einem Flottenverhältnis von 1:50 behandelt.

Die Bedingungen für die in der Tabelle I aufgeführten Sterilisationsverfahren sind im folgenden zusammengestellt:

| Dampfsterilisation: | Gesättigter, gespannter Wasserdampf | | |
|---|---|---|---|
| | Temperatur | : | $134^{\circ}$C |
| | Zeit | : | mind. 10 min |
| Hitzesterilisation : | Heißluft | | |
| | Temperatur | : | $180^{\circ}$C |
| | Zeit | : | 30 min |
| Gassterilisation : | Ethylenoxid | | |
| | Druck | : | 70 Torr |
| | Zeit | : | 90 min |
| Strahlensterilisation : | $\gamma$-Strahlen | | |
| | Dosis | : | 2,5 Mrad |

Grundsätzlich eignet sich ein kohäsiver Verbandstoff der beschriebenen Art je nach Typ des zugrunde liegenden Substrats (Gewebe, Gewirke, Gestricke, Vlies) zur Anwendung als Fixier-, Kompressions-, Stütz- und Entlastungsverband.

Die unter Verwendung von Acrylharzdispersionen hergestellten Verbandstoffe und Binden eignen sich wegen ihrer Beständigkeit gegenüber den bekannten Sterilisationsverfahren, insbesondere zur direkten Anwendung im Sterilbereich. Es ist hiermit möglich, direkt aus einer entsprechenden Verpackung heraus eine sterile Binde für die verschiedensten Anwendungen im Bereich der Wundversorgung, Fixierverbände, Kompressionsverbände, Stütz- und Entlastungsverbände anzulegen.

Die sterilisierten Binden der erfindungsgemäßen Ausgestaltung können jahrelang im verpackten Zustand in Krankenhäusern, Ambulanzen, Apotheken, 1. Hilfestationen und selbst in Verbandkästen gelagert werden, ohne daß es zu einer Beeinträchtigung der Beschaffenheit und der Gebrauchseigenschaften, insbesondere des kohäsiven Klebeverhaltens und optischen Aussehens kommt.

Auch durch eine Lagerung bei drastisch erhöhten Temperaturen, wie z.B. in tropischen Ländern oder bei großer Hitze im Kofferraum eines PKW's, werden die Gebrauchseigenschaften einer solchen Binde in keinster Weise negativ beeinflußt.

Je nach gewünschtem, kohäsivem Effekt und weiteren Gebrauchseigenschaften, wie Luftdurchlässigkeit, Wasserdampfdurchlässigkeit, Saugfähigkeit, elastischem Verhalten und Haftvermögen, stehen verschiedene Auftragstechnologien zur Verfügung, wobei eine vollflächige oder partielle Beschichtung erreicht werden kann.

Anhand der nachfolgenden Beispiele wird die Erfindung erläutert:

Beispiel 1:

Eine baumwollelastische Kompressionsbinde vom Typ Idealbinde DIN 61 632 wird auf einer Walzenrakel mit nachfolgender verschäumter Acrylharzdispersion beschichtet:

1.000 g   Polyacrylsäurebutylester vernetzt (50%ige wäßrige Dispersion)
2 g   Alkylphenylpolyglykoläther (Netzmittel)
10 g   Acrylharzschlichte (Schaumstabilisator)

Die Masse wird in einem Schaummixer im Verhältnis 1:5 verschäumt (Schaumgewicht: 200 g/Liter).

Die Spalthöhe des Rakels wird so eingestellt, daß 20 g Schaum/m² Gewebe einseitig aufgebracht wird. Die Trocknung erfolgt bei 130° C, wobei der Schaum zerfällt und eine vollflächige, mikroporöse Oberflächenbedeckung entstehen läßt. Die Beschichtung der Geweberückseite erfolgt in einem zweiten Arbeitsgang analog. Ein in dieser Weise beschichtetes Gewebe weist folgende physikalisch-techn. Daten auf:

TABELLE II

| | unbeschichtet | beschichtet nach Beispiel 1 | beschichtet nach Beispiel 1 und Strahlensterilisation von 2,5 Mrad. |
|---|---|---|---|
| $m^2$-Gewicht gedehnt | 145 g | 155 g | 155 g |
| Dehnbarkeit | 100 % | 90 % | 90 % |
| Luftdurchlässigkeit *) | 800 l/m$^2$ sek. | 580 l/m$^2$ sek. | 570 l/m$^2$ sek. |
| Haftkraft | / | 78 cN/cm | 74 cN/cm |
| Auflagemenge/m$^2$ | / | 20 g Festsubstanz | 20 g Festsubstanz |

*) nach DIN 53887

Beispiel 2:

Eine ca. 90 g/m² Flächengewicht aufweisende längselastische Fixierbinde wird beidseitig mit ca. 8 g/m²/Seite Dispersion, mikropunktuell nach dem Aerosolverfahren beschichtet.

Rezeptur:

1.000 g    Polyacrylsäurepropylester unvernetzt (60%ige wäßrige Dispersion)

Die wäßrige Dispersion wird unverdünnt nach dem Airlessverfahren verdüst und mikropunktuell auf die Warenbahn in statistischer Verteilung aufgebracht. Die Auflagemenge wird über Pumpendruck und Fördermenge so gewählt, daß pro Gewebeseite eine Auflagemenge von 8 - 10 g/m² Dispersion kommt. Dieses Verfahren hat den Vorteil, daß das elastische Verhalten kaum negativ beeinflußt wird.

TABELLE III

| | unbeschichtet | beschichtet nach Beispiel 2 | beschichtet nach Beispiel 2 und Dampfsterilisation bei 134°C und mind. 5 min. |
|---|---|---|---|
| $m^2$-Gewicht gedehnt | 30 g | 33 g | 33 g |
| Dehnbarkeit | 200 % | 180 % | 180 % |
| Luftdurchlässigkeit *) | 6000 l/m$^2$ sek. | 5800 l/m$^2$ sek. | 5800 l/m$^2$ sek. |
| Haftkraft | / | 38 cN/cm | 40 cN/cm |
| Auflagemenge/m$^2$ | / | 10,5 g Festsubstanz | 10 g Festsubstanz |

*) nach DIN 53887

Beispiel 3:

Mikropunktueller Auftrag durch Siebdruck. Eine ca. 350 g/m²Flächengewicht aufweisende längselastische Kompressionsbinde mit dauerelastischen Polyurethanfäden wird beidseitig mit ca. 15 g/m²/Seite Dispersion durch Siebdruck mikropunktuell kohäsiv beschichtet.

9

Rezeptur:

100 Teile Polyacrylsäurebutylester unvernetzt (55%ige wäßrige Dispersion)
1 Teil Carboxymethylcellulose.

Die verdickte wäßrige Acrylharzdispersion wird unter Verwendung beispielsweise einer 25 mesh Schablone (d.h. 25 Löcher je Zoll linear) mit Lochmusterstruktur auf die Warenbahn aufgedruckt, so daß ein geordnetes Punktmuster auf der Warenoberfläche entsteht. Nach dem Trocknen der beschichteten Warenbahn wird in gleicher Weise die Rückseite des Gewebes mit der beschriebenen Acrylharzdispersion belegt.

T A B E L L E IV

| | unbeschichtet | beschichtet nach Beispiel 3 | beschichtet nach Beispiel 3 und ETO-Sterilisation |
|---|---|---|---|
| $m^2$-Gewicht gedehnt | 165 g | 175 g | 175 g |
| Dehnbarkeit | 100 % | 90 % | 90 % |
| Luftdurchlässigkeit *) | 650 $l/m^2$ sek. | 520 $l/m^2$ sek. | 530 $l/m^2$ sek. |
| Haftkraft | / | 97 cN/cm | 108 cN/cm |
| Auflagemenge /$m^2$ | / | 16 g Festsubstanz | 16 g Festsubstanz |

*) nach DIN 53887

Selbstverständlich ist es möglich, je nach gewünschter Oberflächenstruktur bzw. Beschichtungsmustern, durch Verwendung verschiedener Schablonen die Acrylharzdispersion nach dem Siebdruckverfahren aufzubringen. In dieser Weise ist es möglich, vollflächige Beschichtungen, partielle Beschichtungen nach einem geordneten oder ungeordneten statistischen Punktmuster, geometrische Figuren (Streifen, Gitter) auf die Gewebeoberfläche aufzubringen.

In gleicher Weise ist es möglich, anstelle einer Druckpaste eine verschäumte Acrylharzdispersion, analog Beispiel 1, nach dem Siebdruckverfahren aufzubringen. Dem Fachmann ist es möglich, die beschriebnen Acrylharzdispersionen durch weitere bekannte Auftragsverfahren, wie z.B. Flatschen, Tauchen, Rotationsspritzverfahren, aufzutragen, um Verbandstoffe mit gleichen oder ähnlichen Eigenschaften zu erhalten.

Im folgenden wird der Gegenstand der Erfindung in den Zeichnungen erläutert. Es zeigt

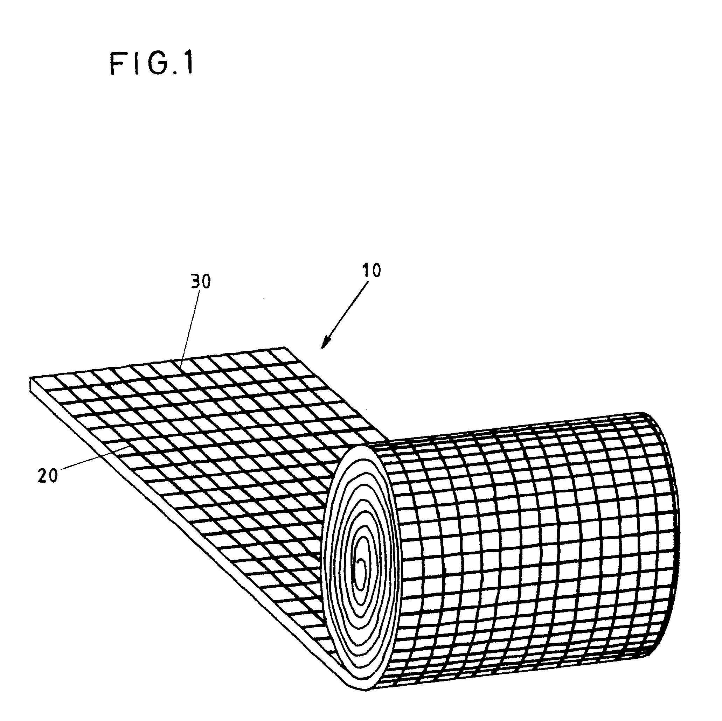

Fig. 1 in einer schaubildlichen Ansicht eine aus dem Verbandstoff hergestellte Binde, teilweise in zusammengerolltem Zustand und

Fig. 2 die Verwendung der kohäsiv ausgestalteten Binde als Stützverband am Unterarm bzw. Ellenbogen.

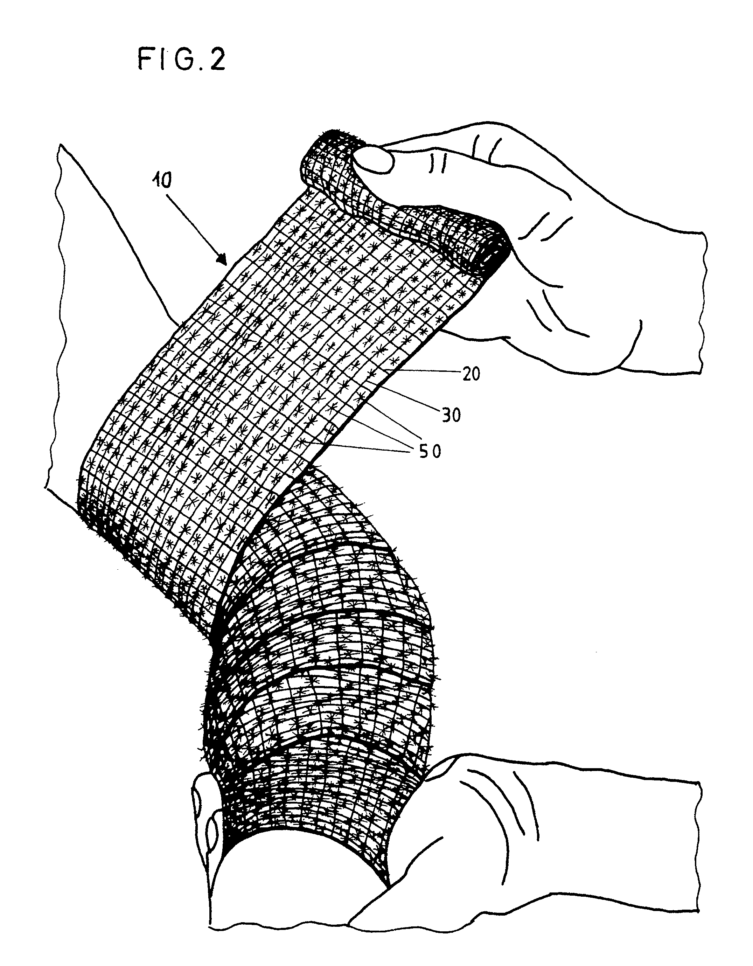

Die aus einem Flächengebilde 10 bestehende und aus dem Verbandstoff hergestellte Binde weist Schußfäden 20 und Kettfäden 30 auf. Beidseitig auf den frei liegenden Oberflächen der Schuß- und Kettfäden 20,30 ist die kohäsive Klebemasse mit Hilfe des Siebdruckverfahrens nach einem Gittermuster aufgebracht, wobei die Klebemassenbereiche bei 50 angedeutet sind (Fig. 2). Die Bindenlagen haften ausschließlich an den Stellen, an denen die Bindentouren aufeinander zu liegen kommen. Auch in den Fällen, in denen es erforderlich sein könnte, kann die Klebemasse auch nur einseitig auf das Flächengebilde 10 aufgebracht sein.

**Ansprüche**

1. Verwendung einer ein- oder beidseitig auf ein Flächengebilde (10) aus einem Gewebe, Gewirke, Gestricke oder Vlies zur Herstellung eines kohäsiven Verbandstoffes, dessen einzelne Lagen oder Bindentouren nur auf sich selbst und nicht an der Haut, Haaren oder Kleidungsstücken haften, für Fixier-, Kompressions-, Stütz- und Entlastungsverbände für medizinische Zwecke, aufgebrachten Klebemasse aus einem im Aerosolverfahren, durch Verdüsung nach dem Airlessverfahren aufbringbare wässrige Di spersion eines Acrylharzes aus der Gruppe der vernetzten oder unvernetzten Polyacrylsäureester zur Erzielung einer Sterilisierbarkei t, A lterungsbeständigkeit und Farbbeständigkeit des Verbandstoffes.

2. Verwendung einer Klebemasse nach Anspruch 1, dadurch gekennzeichnet, daß sie aus einer wässrigen Dispersion aus einem Acrylharz mit einem Netzmittel und/oder Schaumstabilisatoren besteht.

3. Verwendung einer Klebemasse nach Anspruch 1 und 2, dadurch gekennzeichnet, daß sie aus einem Polyacrylsäurebutylester besteht.

4. Verwendung einer Klebemasse nach Anspruch 1 und 2,dadurch gekennzeichnet, daß sie aus einem Polyacrylsäurepropylester besteht.

5. Verwendung einer Klebemasse nach Anspruch 1 bis 3, dadurch gekennzeichnet, daß sie aus 1000 g vernetztem Polyacrylsäurebutylester in
Form einer 50%igen wässrigen Dispersion,
    2 g    Alkylphenylpolyglykoläther als Netzmittel und
    10 g    Acrylharzschlichte als Schaumstabilisator, besteht .

6. Verwendung einer Klebemasse nach Anspruch 1 bis 3, dadurch gekennzeichnet, daß sie aus
    100 Teilen    unvernetztem Polyacrylsäurebutylester in Form einer 50%igen wässrigen Dispersion und
    1 Teil    Carboxymethylcellulose besteht.

7. Verwendung einer Klebemasse nach Anspruch 1,2 und 4, dadurch gekennzeichnet, daß sie aus 1000 g unvernetztem Polyacrylsäurepropylester in Form einer 60%igen wässrigen Dispersion besteht.

8. Verwendung einer beidseitig auf ein Flächengebilde (10) aus einem 90g/m$^2$ Flächengewicht aufweisen-den Gewebe, Gewirke, Gestricke oder Vlies, insbesondere längselastischen Fixierbinde zur Herstellung eines kohäsiven Verbandstoffes, dessen einzelne Lagen oder Bindentouren nur auf sich selbst und nicht an der Haut, Haaren oder Kleidungsstücken haften, für Fixier-, Kompressions-, Stütz- und Entlastungsverbände für medizinische Zwecke, aufgebrachten Klebemasse aus einem im Aerosolverfahren, durch Verdüsung nach dem Airlessverfahren mit 8g/m$^2$/Seite auf-bringbare 60%ige wässrige Dispersion aus 100g unvernetztem Polyacrylsäurepropylester zur Erzielung einer Sterilisierbarkeit, Alterungsbeständigkeit und Farbbeständigkeit des Verbandstoffes.

## Claims

1. Use of an adhesive applied to one side or both sides of a flat fabric (10) comprised of a woven cloth, finely or coarsely knitted fabric or of a non-woven for the production of a cohesive dressing, the individual layers or bandaging turns of which only adhere to themselves and not to the skin, hair or garments, for immovable, compression, suspensory and relief dressings for medical purposes, said adhesive being an aqueous dispersion of an acrylic resin from the group of the crosslinked or non-cross linked polyacrylic acid esters applicable by the aerosol method, by atomization according to the airless method in order to achieve a sterilizability, resistance to aging and color fastness of the dressing.

2. Use of an adhesive according to Claim 1, characterized in that it is comprised of an aqueous solution of an acrylic resin with a wetting agent and/or foam stabilizers.

3. Use of an adhesive according to Claims 1 and 2, characterized in that it is comprised of a polyacrylic acid butyl ester.

4. Use of an adhesive according to Claims 1 and 2, characterized in that it is comprised of a polyacrylic acid propyl ester.

5. Use of an adhesive according to Claims 1 to 3, characterized in that it is comprised of 1000 g crosslinked polyacrylic acid butyl ester
in the form of a 50% aqueous dispersion,

    2 g     alkylphenyl polyglycol ether as wetting agent and
    10 g    acrylic resin size as foam stabilizer.

6. Use of an adhesive according to Claims 1 to 3, characterized in that it is comprised of

    100 parts    non-crosslinked polyacrylic acid butyl ester in the form of a 50% aqueous dispersion and
    1 part       carboxymethylcellulose.

7. Use of an adhesive according to Claims 1,2 and 3, characterized in that it is comprised of 1000 g non-crosslinked polyacrylic acid propyl ester in the form of a 60% aqueous dispersion.

8. Use of an adhesive applied to both sides of a flat fabric (10) comprised of a woven cloth, finely or coarsely knitted fabric or of a non-woven possessing 90g/m² weight per unit area, more particularly of a longitudinally elastic immovable bandage for the production of a cohesive dressing, the individual layers or bandaging turns of which only adhere to themselves and not to the skin, hair or garments, for immovable, compression, suspensory and relief dressings for medical purposes, said adhesive being a 60% aqueous dispersion of 100 g non-crosslinked polyacryli c acid propyl ester applicable by the aerosol method, by atomization according to the airless method at 8g/m²/side in order to achieve a sterilizability, resistance to aging and color fastness of the dressing.

## Revendications

1. Utilisation d'un adhésif appliqué d'un côté ou des deux côtés sur une structure de surface (10) constituée par un tissu, un tissu à mailles, un tricot ou un non-tissé, pour fabriquer un pansement cohésif dont les différentes couches ou tours de bandage n'adhèrent que sur eux-mêmes et non pas à la peau, aux cheveux ou aux vêtements, pour des pansements de fixation, de compression, de support et de décompression à usages médicaux, adhésif constitué par une dispersion aqueuse d'une résine acrylique du groupe des polyesters acryliques réticulés ou non réticulés pouvant être appliquée en technique aéro-sol par atomisation selon le procédé airless pour obtenir la stérilisabilité, la résistance au vieillissement et la stabilité des couleurs du pansement.

2. Utilisation d'un adhésif selon la revendication 1, caractérisée en ce que l'adhésif est constitué par une dispersion aqueuse d'une résine acrylique avec un agent mouillant et/ou des stabilisateurs de mousse.

3. Utilisation d'un adhésif selon les revendications 1 et 2, caractérisée en ce que l'adhésif est constitué par un polyacrylate de butyle.

4. Utilisation d'un adhésif selon les revendications 1 et 2, caractérisée en ce que l'adhésif est constitué par un polyacrylate de propyle.

5. Utilisation d'un adhésif selon les revendications 1 à 3, caractérisée en ce que l'adhésif est constitué par 1000g de polyacrylate de butyle réticulé
sous forme d'une dispersion aqueuse à 50%,

    de 2 g     de polyglycoléther alkylophénylique comme agent mouillant et
    de 10 g    de produit d'encollage à base d'acide acrylique comme stabilisateur de mousse.

6. Utilisation d'un adhésif selon les revendications 1 à 3, caractérisée en ce que l'adhésif est constitué par 100 parts de polyacrylate de butyle non réticulé sous forme d'une dispersion aqueuse à 50% et par 1 part de cellulose carboxéthylique.

7. Utilisation d'un adhésif selon les revendications 1,2 et 4, caractérisée en ce que l'adhésif est constitué par 1000g de polyacrylate de propyle non réticulé sous forme d'une dispersion aqueuse à 60%.

8. Utilisation d'un adhésif appliqué d'un côté ou des deux côtés sur une structure de surface (10) constituée par un tissu, un tissu à mailles, un tricot ou un non-tissé présentant un grammage de 90 g/m², en particulier un bandage de fixation élastique en longueur, pour fabriquer un pansement cohésif dont les différentes couches ou tours de bandage n'adhèrent que sur eux-mêmes et non pas à la peau, aux cheveux ou aux vêtements, pour des pansements de fixation, de compression, de support et de décompression à usages médicaux, adhésif constitué par une dispersion aqueuse à 60% de 100 g de polyacrylate de propyle non réticulés pouvant être appliquée en technique aéro-sol par atomisation selon le procédé airless avec 8 g/m² pour obtenir la stérilisabilité, la résistance au vieillissement et la stabilité des couleurs du pansement.

FIG.1

# FIG.2